# EUROPEAN PATENT SPECIFICATION

(11) **EP 1 852 130 B1**
(45) Date of publication and mention of the grant of the patent: **20.06.2012**
(21) Application number: 06714571.4
(22) Date of filing: 24.02.2006
(51) Int. Cl.: A61K 47/24, A61K 9/70, A61K 31/192, A61P 29/00

(54) **MEDICINE FOR EXTERNAL APPLICATION CONTAINING ANTI-INFLAMMATORY AGENT AND SOY LECITHIN**
MEDIZIN ZUR ÄUSSEREN ANWENDUNG MIT ENTZÜNDUNGSHEMMENDEM MITTEL UND SOJALECITHIN
MEDICAMENT POUR APPLICATION EXTERNE CONTENANT UN AGENT ANTI-INFLAMMATOIRE ET DE LA LECITHINE DE SOJA

(30) Priority: 25.02.2005 JP 2005050994
(43) Date of publication of application: 07.11.2007
(73) Proprietor: HISAMITSU PHARMACEUTICAL CO., INC., Tosu-shi, Saga-ken 841-0017 (JP)
(72) Inventor: KOZUMA, Miyuki, rodaikanmachi, Tosu-shi, Saga, 8410017 (JP); TANAKA, Koji, rodaikanmachi, Tosu-shi, Saga, 8410017 (JP); HASHIMOTO, Yoshiaki, rodaikanmachi, Tosu-shi, Saga, 8410017 (JP); SUZUKI, Shigeo, rodaikanmachi, Tosu-shi, Saga, 8410017 (JP)
(74) Representative: Forstmeyer, Dietmar
(86) International application number: PCT/JP2006/303432
(87) International publication number: WO 2006/090839

(56) References cited:
- WO-A1-01/68061
- JP-A- 59 055 819
- JP-A- 60 155 111
- JP-A- 2000 327 570
- JP-A- 2004 231 600
- US-B1- 6 437 002
- MAHJOUR M ET AL: "EFFECT OF EGG YOLK LECITHINS AND COMMERCIAL SOYBEAN LECITHINS ON IN VITRO SKIN PERMEATION OF DRUGS" JOURNAL OF CONTROLLED RELEASE, ELSEVIER, AMSTERDAM, NL, vol. 14, no. 3, 1 December 1990 (1990-12-01), pages 243-252, XP000165642 ISSN: 0168-3659

## Description

### [Technical Field]

The invention relates to an external application, which enables the use of ketoprofen with better safety.
More particularly, the invention relates to the external application, in which the penetration of an organic ultraviolet absorbent to skin is enhanced by soy lecithin and the onset of photo-allergies caused by ketoprofen is suppressed by the organic ultraviolet absorbent.

### [Background Art]

An anti-inflammatory agent is frequently used for the therapy and mitigation of the inflammation in muscular pain and lower-back pain, and it is used in a wide range of dosage forms such as an oral preparation, an injection, and an external application. Among these, when the anti-inflammatory agent is used for external application, an excessive exposure to light such as sunlight causes, even though rarely, contact dermatitis, photosensitivity and the like.
Approaches to suppress photosensitivity have been reported, for example, blending of a UV absorber into a preparation for external application, as an attempt to suppress the formation of photodecomposition products by preventing photodecomposition of the anti-inflammatory agent (ref. Patent document 1); application of a UV blocking treatment to the backing of the patch containing a nonsteroidal anti-inflammatory analgesic (ref. Patent document 2); and blending of titanium oxide into an anti-inflammatory medicine for external application to the skin (ref. Patent document 3). However, a preparation to further facilitate the preventive effect on photosensitivity and the like has been desired.

Patent document 1: JP, A, 60-155111
Patent document 2: WO 01/68061
Patent document 3: JP, A, 9-169658
Patent document 4: JP, A, 53-99316
Patent document 5: JP, A, 56-22711
Patent document 6: JP, A, 2000-136122

JP 2000-327570 discloses a skin preparation for external use comprising urea, a phosphoryl choline-like group containing polymer, a fat or oil containing one or more kinds of particular fatty acids and a masking agent.

Mahjour et al., Journal of Controlled Release (1990), 14, 243-252 disclose skin permeation enhancement effects of egg yolk lecithins and commercial soy bean lecithins on in vitro skin permeation of drugs.

JP 59-55819 relates to a plaster for adhesive salve preparation comprising a rubber-based adhesive material and lecithin, especially soy bean lecithin or yolk lecithin. The lecithin additive is effective to prevent decomposition of the active agent in the plaster.

### [Disclosure of the Invention]

### [Problems to Be Solved by the Invention]

The invention makes it an object to more surely prevent the photosensitivity caused by a transdermal preparation for an external application containing a photosensitive anti-inflammatory agent.

### [Means to solve the problems]

During an extensive research to attain the object above, the inventors noticed that when an organic ultraviolet absorbent is blended into an external application, photo-allergies or the like caused by ketoprofen can sufficiently be suppressed by the transference of the organic ultraviolet absorbent to the skins, and that the photo-allergies and the like can efficiently be prevented by improving the penetration of the organic ultraviolet absorbent to skin while not improving the transdermal absorption of the drug.
The inventors continued further research based on such notice, and consequently found that soy lecithin increases transference of the organic ultraviolet absorbent to the skin while does not increase a penetration of ketoprofen to skin, and accordingly accomplished the invention.

Namely, the invention relates to an external application containing ketoprofen, an organic ultraviolet absorbent selected from a group consisting of an external application, containing ketoprofen, an organic ultraviolet absorbent and soy lecithin, wherein the organic ultraviolet absorbent is selected from a group consisting of benzoylmethane derivative, benzophenone derivative, cinnamic derivative, camphor derivative, benzotriazole derivative, amino compounds, and a benzoylpinacolone derivative, and soy lecithin.
In addition, the invention relates to the external application as a patch, in which a backing is laminated to an adhesive base.
In addition, the invention relates to the external application, wherein the organic ultraviolet absorbent is 4-tert-butyl-4' -methoxydibenzoylmethane.
Furthermore, the invention relates to the external application, wherein the ratio of ketoprofen : the organic ultraviolet absorbent: soy lecithin is 2 : 3-4 : 1~2.

### [Effect of the Invention]

In the external application of the invention, the onset of photo-allergies, such as photosensitivity, caused by ketoprofen is remarkably suppressed, and the anti-inflammatory analgesic effect can sufficiently be exerted; accordingly, the application as a medicine with extremely high safety is expected.
In the external application of the invention, the mechanism is not necessarily apparent, by which soy lecithin increases the transference of an organic ultraviolet absorbent to skin. However, it is considered that the surfactant action of lecithin increases the distribution of the organic ultraviolet absorbent, which has an extremely high lipophilicity, to the water-containing corneum.

### [Most Preferred Embodiment of the invention]

In the following, the external application of the invention is explained in more detail.
The external application of the invention is not particularly limited in its dosage form. Examples include applications such as a patch, a cream, an ointment and a lotion, an aerosol preparation and the like, however, a patch, which stays fit onto the body over time and can provide a drug over the time course, in preferable.
The patch is constituted with an adhesive base and a backing laminated onto it.

The external application of the invention constains ketoprofen, an organic ultraviolet absorbent, and soy lecithin.
The blending amount is not particularly limited as long as it shows a sufficient drug effect, however, it is blended in 0.1 % by mass to 10 % by mass, preferably in 0.2 % by mass to 5 % by mass.

The organic ultraviolet absorbent blended in the external application of the invention and is selected from a group consisting of benzoylmethane derivatives, benzophenone derivatives, cinnamic derivatives, camphor derivatives, benzotriazole derivatives, amino compounds, and a benzoylpinacolone derivatives. More preferably, it is selected from a group consisting of 4-tert-butyl-4' -methoxydibenzoylmethane, n-hexyl 2-(4-diethylamino-2-hydroxybenzoyl)benzoate, 4-hydroxy-3-methoxycinnamic acid, a branched alkyl ester of 4-hydroxy-3-methoxycinnamic acid, terephthalylidene-3.3'-dicamphor-10,10'-disulfonic acid, 2-(2H-benzotriazol-2-yl)-4-methyl-6-[2-methyl-3-[1,3,3,3-te tramethyl-1-[(trimethylsilyl)oxy]disiloxanyl]propyl]phenol, 2-hexyl dimethoxybenzylidenedioxoimidazolidinepropionate, 1-(3,4-dimthoxyphenyl)-4,4-dimethyl-1,3-pentanedione; more preferably, 4-*tert*-butyl-4' -methoxydibenzoylmethane (hereinafter, BM-DBM), and similar compounds are included as the ultraviolet absorbent; in particular, 4-*tert*-butyl-4'-methoxydibenzoylmethane is preferable.
Although the blending amount of the organic ultraviolet absorbent is not particularly limited as long as it shows a favorable reducing effect on photo-allergies, it is blended in 0.01 % by mass to 20 % by mass, preferably 1 % by mass to 10 % by mass.

In addition, since BM-DBM is in crystalline state at ordinary temperature, it is preferable to simultaneously blend a solvent for BM-DBM in order to be blended into the adhesive base of the patch. As such solvent, normally usable solvents can appropriately be selected, such as, for example, crotamiton, diethyl sebacate, diisopropyl sebacate, diisopropyl adipate, soy lecitin, hexyl laurate, propylene glycol monocaprylate, propylene glycol dicaprylate
Although the blending amount of the solvent is not particularly limited, it is blended in 0.1 % by mass to 10 % by mass, preferably 1 % by mass to 5 % by mass, considering physical properties of a preparation.

Soy lecithin blended in the external application of the invention is one of phospholipid, consisting of saturated fatty acids such as palmitic acid and stearic acid, and unsaturated fatty acids oleic acid and linoleic acid, etc.
This soy lecithin can be blended in 0.1 % by mass to 5 % by mass, more preferably 1 % by mass to 2 % by mass in a preparation, considering physical properties of the preparation, particularly in case of a plaster, the adhesiveness of the plaster to akin.

When the amount of the organic ultraviolet absorbent transferred to skin is not sufficient, photo-allergies due to the ketoprofen tend not surely be inhibited. In the external application of the invention, soy lecithin is contained, and therefore, the transference of the organic ultraviolet absorbent to skin in increased and the transdermal absorption amount of the ketoprofen is not increased. Therefore, the external application according to the invention sufficiently inhibits the onset of photo-allergies and the like caused by photodecomposition of ketoprofen, and can sufficiently exert an anti-inflammatory action usually desired.

The blend ratio of ketoprofen : the organic ultraviolet absorbent: soy lecithin in the external application of the invention 2:3~4:1~2.
It is not preferable that the blend ratio of the organic ultraviolet absorbent is at the lower limit of said range or less, since the obtained inhibitory effect on photo-allergies tends to be insufficient. The upper limit or over is not quite preferable, since crystallization tends to occur in the preparation.
In addition, it is not preferable that the blend ratio of say lecithin is at the lower limit of said range or less, since the transference of the organic ultraviolet absorbent to the corneum tends not be increased. The ratio at the upper limit or over is neither preferable, since the physical properties of the preparation tend to be changed, for example, in the case of a plaster, to reduce the adhesive force.

In the case that the dosage form of the external application of the invention is a patch, the adhesive base is not particularly limited as long as it is an adhesive base conventionally used in a patch; however, a rubber type, an acrylic type, a silicone type, or a mixture of two or more of these can be used.
The rubber base is not particularly limited, and is selected from a polyisoprene rubber such as a synthetic rubber or a natural rubber, a styrene-butadiene copolymer, a styrene-isoprene copolymer, a styrene-isoprene-styrene block copolymer, a styrene-butadiene-styrene block copolymer, polyisobutylene and the like.
Although the acrylic adhesive base is not particularly limited, a copolymer between alkyl(meth)acrylate, which is obtained from a C₄-C₁₈ aliphatic alcohol and (meth)acrylic acid, and vinylpyrrolidone or other functional monomers are suitable.

The acrylate polymer is not particularly limited as long as its weight-average molecular weight is not less than 2,500,000 and it can be dissolved with a liquefied plasticizer mentioned below; however, it is preferably a copolymer in which alkyl(meth)acrylates are copolymerized as a main component. The alkyl(meth)acrylate includes the alkyl (meth)acrylate in which, specifically, alkyl groups are a straight chain alkyl group or a branched alkyl group with a carbon number of 4 or more, such as butyl, pentyl, hexyl, heptyl, octyl, nonyl, decyl, undecyl, dodecyl, tridecyl, used either alone or in combination of two or more.

As a monomer which can be copolymerized with the said alkyl (meth)acrylate, examples include monomers having a carboxyl group such as (meth)acrylic acid, itaconic acid, maleic acid and maleic anhydride; monomers having a sulfonyl group such as styrenesulfonic acid, allyl sulfonic acid, sulfopropyl (meth)acrylate, (meth)acryloyloxynaphthalenesulfonic acid and acrylamidomethylpropanesulfonic acid; monomers having a hydoxyl group such as hydroxyethyl (meth)acrylate and hydoxylpropyl (meth)acrylate; (meth)acrylic acid derivatives having an amido group such as (meth)acrylamide, dimethyl(meth)acrylamide, N-butyl(meth)acrylamide and N-methylol(meth)acrylamide; aminoalkyl (meth)acrylates such as aminoethyl (meth)acrylate dimethylaminoethyl (meth)acrylate and t-butylaminoethyl (meth)acrylate; alkoxyalkyl (meth)acrylates such as methoxyethyl (meth)acrylate, ethoxyethyl (meth)acrylate and tetrahydrofurfuryl (meth)acrylate; alkoxyalkylene glycol (meth)acrylate such as methoxyethylene glycol (meth)acrylate, methoxydiethylene glycol (meth)acrylate, methoxypolyethylene glycol (meth)acrylate and methoxypolypropylene glycol (meth)acrylate; compounds having a vinyl group such as (meth)acrylonitrile, vinyl acetate, vinyl propionate, N-vinyl-2-pyrrolidone, methylvinyl pyrrolidone, vinylpyridine, vinylpiperidine, vinylpyrimidine, vinylpiperazine, vinylpyrazine, vinylpyrrole, vinylimidazole, vinylcaprolactam, vinyloxazole and vinylmorpholine. These are used either alone or in combination of two or more. The copolymerizing amount of these monomers is appropriately set according to a weight average molecular weight of the copolymer to be obtained.
As an adhesive base of silicone type, polydimethylsiloxane is preferable.

The blending amount of these polymers used in the adhesive base based on the weight of the total composition is 5-50 % by mass, preferably 20-40 % by mass, more preferably 25-35 % by mass considering the formation of the adhesive layer and a sufficient penetration.
In the adhesive base, a tackifier, a plasticizer, a filler, an absorption promoter and the like are appropriately blended.
As a tackifier, those with softening point of 60°C-150°C are preferable; for example, rosin esters, hydrogenated rosin esters, maleic acid degenerated rosin esters, polyterpene resins and petroleum resins can be used.

Plasticizers include petroleum oils (*e.g.,* paraffin type process oil, naphthene type process oil, aromatic type process oil, etc.), squalane, squalene, vegetable oils (*e.g*., olive oil, camellia oil, castor oil, tall oil, peanut oil), silicone oil, dibasic acid esters (e.g. , dibutyl phthalate, dioctyl phthalate, etc.), liquefied rubbers (*e.g*., polybutene, liquefied isoprene rubber), liquefied fatty acid esters (*e.g*., isopropyl myristate, hexyl laurate, diethyl sebacate, diisopropyl sebacate), diethylene glycol, polyethylene glycol, glycol salicylate, propylene glycol, dipropylene glycol, triacetin , trietyl citrate, crotamiton, and the like. In particular, liquid paraffin, liquefied polybutene, isopropyl myristate, diethyl sebacate, hexyl laurate and the like can be used.

As fillers, calcium carbonate, magnesium carbonate, silicates (*e.g*., aluminum silicate, magnesium silicate, etc.), silicic acid, barium sulfate, calcium sulfate, calcium zincate, zinc oxide, titanium oxide, and the like can be used.

Although the external application of the invention can be produced by any production methods known to those skilled in the art, in the following production of a patch is explained as an example.
The pressure-sensitive adhesive layer containing drugs can be produced by any method; however, for example, the drug-containing base composition is heat-melted, then applied onto an removable paper or a backing, which is then affixed to the backing or the removable paper to give the preparation. Alternatively, base ingredients containing the drug are dissolved in a solvent such as toluene, hexane, or ethyl acetate, spread on the removable paper or the backing, dried to remove the solvent, then affixed to the backing or the removable paper to give the patch.

As for the backing of the patch of the invention, a stretch or non-stretch backing may be used. For example, it is selected from woven fabric, knitted fabric, non-woven fabric, polyurethane, polyester, polyvinyl acetate, polyvinylidene chloride, polyethylene, polyethylene terephthalate, aluminum sheet, or a composite material thereof.
In addition, a liner is not particularly limited as long as it can protect the adhesive layer till the patch is applied to the skin; however, specifically, a liner includes a film of polyesters (e.g., polyethylene terephthalate), polyvinyl chloride and polyvinylidene chloride and the like; a laminated film of high-quality paper and polyolefin and the like. In such liner, the side attached to the adhesive layer is preferably siliconized, so that the operation upon peeling off the liner from the preparation is facilitated.

### [Example]

The invention is explained in more detail by the examples below. The invention, however, is not limited to these examples, and various alterations may be made without departing from the spirit of the invention. In addition, in the examples, "%" always means % by mass.

### (Example 1)

Styrene-isoprene-styrene block polymer, polyisobutylene, resin and liquid paraffin were stirred while heated under an atmosphere of nitrogen gas to give a solution. Then, ketoprofen, 1-menthol, 4-*tert*-butyl-4'-methoxydibenzoylmethane, solvent, and soy lecithin were added to the said solution and mixed to give a base for a plaster preparation as a uniform solution. Said base was spread over a silicone-treated polyester film at weight of 1 g per 70 cm², then covered with a polyester woven fabric, transferred by crimping, and cut to a desired size to give a plaster preparation of the invention.
The specific blending amount of each ingredient is shown in Table 1.

### (Example 2)

A plaster preparation was obtained in the same way as that of the example 1 according the formula shown in Table 1.

### (Comparative example 1)

A plaster preparation was obtained in the same way as that of the example 1 according the formula shown in Table 1.

**[Table 1]**

| Ingredients | | | |
|---|---|---|---|
| | Comparative example 1 | Example 1 | Example 2 |
| Styrene-isoprene-styrene block copolymer | 30 | 30 | 30 |
| Liquid paraffin | 36.5 | 35.5 | 34.5 |
| Polyisobutylene | 7.5 | 7.5 | 7.5 |
| Resin | 14 | 14 | 14 |
| Ketoprofen | 2 | 2 | 2 |
| BM-DBM | 3 | 3 | 3 |
| Solvent | 5 | 5 | 5 |
| Soy lecithin | 0 | 1 | 2 |

(Experiment of transferred amount using hairless mouse skin) Experiment of transferred amount of BM-DBM using hairless mouse skin
The skin taken from a hairless mouse by a conventional method was divided into 4 equal parts and spread on a filter paper (7x7cm²) containing 1mL of the physiological saline. A preparation cut in φ 15mm was applied onto the skin, which was then covered with an appropriate vessel and left on a constant-temperature floor set at 35°C. 300µL of purified water was added to the filter paper every 60 minutes from beneath. 6 hours after application, the skin of the hairless mouse was cut out using a cutting knife of φ 20mm so that the preparation became the center. The preparation was slowly peeled off from the skin cut out to make a sample. BM-DBM and ketoprofen were extracted from the skin sample to obtain the intradermal amount of BM-DBM and ketoprofen.

### [Result 1] BM-DBM transfer amount

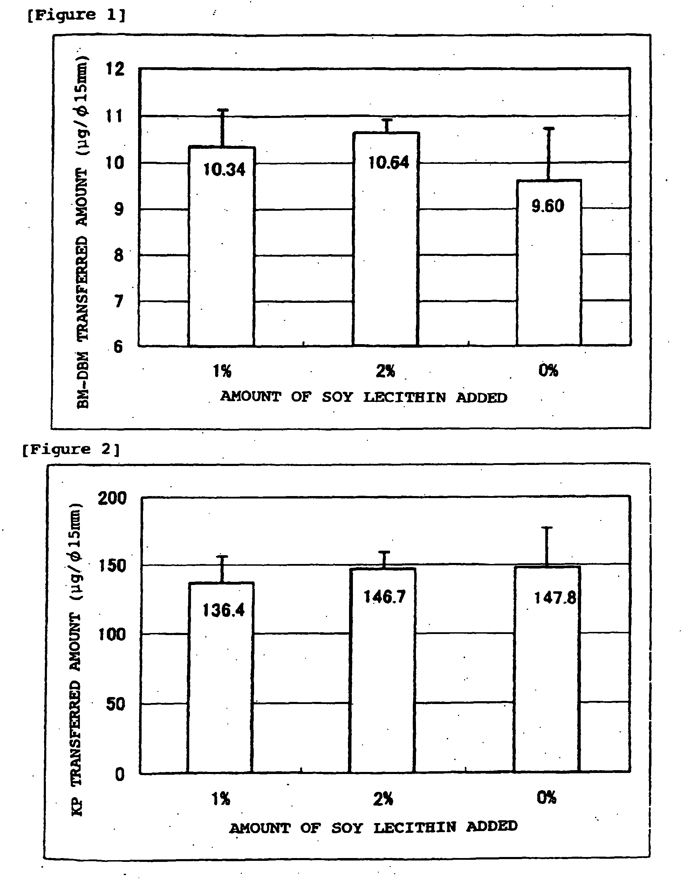

The preparation was prepared with the concentration of soy lecithin set to 0% (comparative example 1), 1% (example 1) and 2% (example 2), and the amount of BM-DBM transferred to the skin was evaluated. The results are shown in Fig.1. It was confirmed that in the patches in which the adding amount of soy lecithin was 1% (example 1) and 2% (example 2) respectively, the amount of BM-DBM transferred was promoted compared with the patch of the comparative example 1 containing no soy lecithin.

**[Table 2]**

| | |
|---|---|
| Example 1 | Resin 14.2%, Soy lecithin 1% |
| Example 2 | Resin 14%, Soy lecithin 2% |
| Comparative example 1 | Resin 14%, Soy lecithin None |

### [Result 2] Ketoprofen transfer amount

The preparations in which the concentration of soy lecithin was set to 0% (comparative example 1), 1% (example 1) and 2% (example 2) were prepared, and the amount of ketoprofen transferred to the skin was evaluated. The results are shown in Fig. 2. It was confirmed that the patches in which the adding amount of soy lecithin was 1% (example 1) and 2% (example 2) respectively, showed the same degree of the skin transfer amount of ketoprofen as that of the patch of the comparative example 1 containing no soy lecithin.

### [Reaction-inducing test using photosensitized guinea pigs]

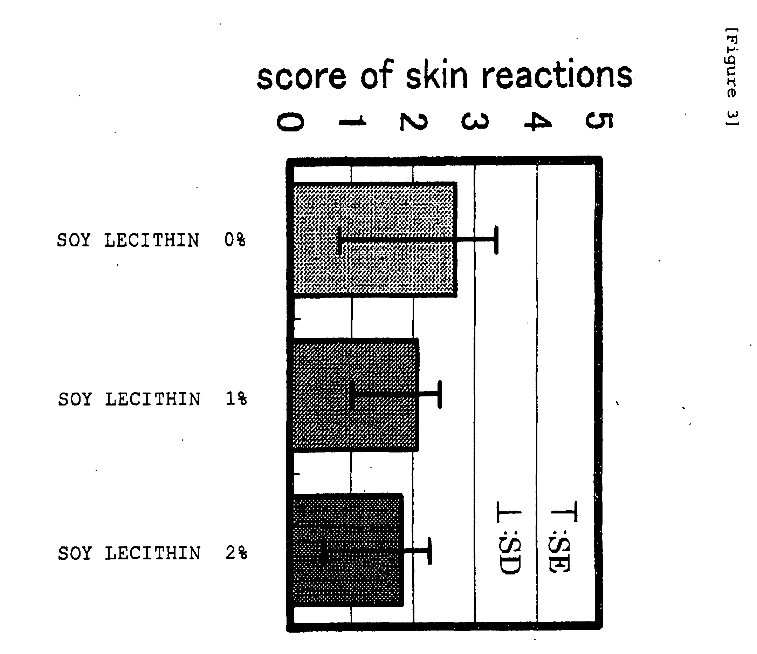

A skin photosensitization test using guinea pigs was carried out by referring to and partly modify the Adjuvant and Strip method by Satoh et al. (Nishinihon Hifuka, 42, 831-837). Namely, the dosal part of the cervix of a female white Hartley strain guinea pig was depilated; the adjuvant was applied to four corners of 2x2cm; a ketoprofen preparation cut in the same size was applied for 4 hours, followed by irradiation of UV-A (10 J/cm2) after peeling off. This sensitization-inducing treatment was carried act successively for 5 days. 3 weeks after the start of the sensitization, the dosal lumber region was depilated, and three preparations cut in the size of 1.5x1.5cm, in which BH-DBM was blended to a ketoprofen preparation and the concentration of soy lecithin was set to 0% (comparative example 1), 1% (example 1) and 2% (example 2), were applied for 4 hours, followed by irradiation of UV-A (2.5 J/cm²) after peeling off to carry out photo-induction. Skin reactions 24 hours after the irradiation were evaluated according to the standard of Satoh et al, by total 7 points (0-4 points for erythema and 0-3 points for edema). The results are shown in Fig. 3.

### (Test result)

In the test, it was observed that the addition of soy lecithin suppresses the skin reaction better compared with BM-DBM only.

### (Industrial applicability]

As explained above, according to the invention, it is enabled to exert anti-inflamnatory analgesic effects while more surely preventing photo-allergies, in a external application containing ketoprofen which is likely to induce photo-allergies, and therefore, its application as a drug with high safety can be expected.

### [Brief Description of drawing]

Figure 1 shows the transferred amount of BM-DBM when the patch of the invention was applied.
Figure 2 shows the transferred amount of ketoprofen when the patch of the invention was applied.
Figure 3 shows the results of the reaction-inducing test when the patch of the invention was applied.

## Claims

1. An external application, containing ketoprofen, an organic ultraviolet absorbent and soy lecithin, wherein the organic ultraviolet absorbent is selected from a group consisting of benzoylmethane derivative, benzophenone derivative, cinnamic derivative, camphor derivative, benzotriazole derivative, amino compounds, and a benzoylpinacolone derivative.

2. The external application according to claim 1, wherein the transference of the organic ultraviolet absorbent to skin is increased and the transdermal absorption amount of ketoprofen is the same degree as that containing no soy lecithin.

3. The external application according to claim 1 or 2 as a patch, in which a backing is laminated to an adhesive layer.

4. The external application according to any one of claims 1 to 3, wherein the organic ultraviolet absorbent is 4-*tert-*butyl-4'-methoxydibenzoylmethane.

5. The external application according to any one of claims 1 to 4, wherein the ratio of ketoprofen : the organic ultraviolet absorbent: soy lecithin is 2:3∼4:1∼2.

## Patentansprüche

1. Externe Applikation, enthaltend Ketoprofen, ein organisches UV-Absorbens und Sojalecithin, wobei das organische UV-Absorbens ausgewählt ist aus einer Gruppe, bestehend aus einem Benzoylmethan-Derivat, Benzophenon-Derivat, Zimtsäure-Derivat, Campher-Derivat, Benzotriazol-Derivat, Aminoverbindungen, und einem Benzoylpinacolon-Derivat.

2. Die externe Applikation nach Anspruch 1, wobei der Transfer des organischen UV-Absorbens in die Haut gesteigert ist und die Menge an transdermal absorbiertem Ketoprofen gleich ist wie bei einer Applikation, die kein Sojalecithin enthält.

3. Die externe Applikation nach Anspruch 1 oder 2 als ein Pflaster, bei dem eine Schutzfolie auf eine Klebschicht laminiert ist.

4. Die externe Applikation nach einem der Ansprüche 1 bis 3, wobei das organische UV-Absorbens 4-*tert*-Butyl-4'-methoxydibenzoylmethan ist.

5. Die externe Applikation nach einem der Ansprüche 1 bis 4, wobei das Verhältnis von Ketoprofen : dem organischen UV-Absorbens : Sojalecithin 2 : 3~4 : 1∼2 ist.

## Revendications

1. Application externe, contenant du kétoprofène, un absorbant d'ultraviolets organique et de la lécithine de soja, où l'absorbant d'ultraviolets organique est sélectionné dans un groupe constitué d'un dérivé de dibenzoylméthane, d'un dérivé de benzophénone, d'un dérivé de l'acide cinnamique, d'un dérivé du camphre, d'un dérivé de benzotriazole, de composés amino, et d'un dérivé de benzoylpinacolone.

2. Application externe selon la revendication 1, dans laquelle le transfert de l'absorbant d'ultraviolets organique à la peau est augmenté et la quantité d'absorption transdermique de kétoprofène est de même degré que celui de l'application ne contenant pas de la lécithine de soja.

3. Application externe selon la revendication 1 ou 2 telle qu'un timbre, où un support est stratifié sur une couche adhésive.

4. Application externe selon l'une quelconque des revendications 1 à 3, dans laquelle l'absorbant d'ultraviolets organique est le 4-tert-butyl-4'-méthoxydibenzoylméthane.

5. Application externe selon l'une quelconque des revendications 1 à 4, dans laquelle le rapport de kétoprofène: l'absorbant d'ultraviolets organique: la lécithine de soja est de 2:3∼4:1∼2.
